# EUROPEAN PATENT APPLICATION

(11) **EP 4 786 575 A1**
(43) Date of publication of application: **05.08.2026**
(21) Application number: 25382055.9
(22) Date of filing: 30.01.2025
(51) Int. Cl.: C12N 1/16, C12N 1/22, C12R 1/645

(54) **OLEAGINUS YEAST AND PRODUCTION OF MICROBIAL OILS**

(71) Applicant: Iberia Bioenergy, S.l.u., 18009 Granada (ES)
(72) Inventor: COBO SEGURA, JUAN MIGUEL, 18199 CÁJAR (GRANADA) ES (ES)
(74) Representative: Veiga Serrano, Mikel

(57) **Abstract**

The present invention relates to a strain of *Rhodosporidium toruloides* and a method for producing microbial oils. The method utilizes organic waste, preferably lignocellulosic sugars derived from almond shell and/or olive pit, as a carbon source. This approach provides an efficient and sustainable method for converting waste into valuable microbial oils, an advanced feedstock with low carbon intensity suitable for the production of sustainable fuels, biolubricants, biosurfactants, and other industrial products.

## Description

### Technical Field

The present invention relates to the production of oils, fuels, and oleochemicals made from oleaginous yeast.

### Background

Climate change, socio-economic pressures and evolving policies are accelerating the global push toward decarbonization, prompting a transition from a fossil fuel-based economy to one rooted in biomass. This paradigm shift requires not only replacing fossil fuels as energy sources, but also moving away from crude oil as the foundation for chemicals, polymers, pharmaceuticals, solvents and numerous other essential materials. To meet the United Nations Sustainable Development Goals, crude oil must be substituted with renewable resources. In this context, biorefineries represent a sustainable alternative to traditional refineries, enabling the production of biofuels, chemicals and bio-based materials from inedible biomass and waste.

Microbial oils are gaining traction as promising substitutes in the oleochemical industry, supporting the shift towards renewable and sustainable production systems. These oils offer significant advantages due to their high quality, low carbon footprint, rapid cultivation, independence from geographical and climatic constraints, reduce land use and non-competition with the food supply. Despite their potential, the large-scale production of microbial oils faces challenges, primarily due to high processing costs and expensive feedstocks. Therefore, new production strategies that leverage combined sustainable residues and waste as feedstocks are critical to reducing costs and enabling the industrial-scale production of microbial oils.

The state of the art describes methods and materials for the production of oils using oleaginous microbes, including yeasts. However, there remains a critical need for improved methods that employ yeasts capable of growing and accumulating high fat content from a wide range of residual feedstocks, including complex mixtures of waste materials.

The present invention addresses this need by utilizing an oleaginous yeast strain specifically suited to accumulate significant quantities of microbial oil using organic waste derived from a diverse range of industrial, municipal and/or lignocellulosic by-products derived from agricultural and forestry sources, thus providing a more efficient and sustainable approach to microbial oil production.

EP2558565A1 discloses the *R. toruloides* strain CBS14 and its use in the production of microbial oils.

WO2009118438A1 discloses an improved process for the production of biodiesel, including a strain of *R. toruloides* CECT 13006 that is capable of achieving a lipid concentration of 53.7% by weight using crude glycerin as carbon source

However, none of these documents disclose the *R. toruloides* of the present invention, neither an adapted method of production of microbial oils from organic waste using said strain.

### Description

A first object of the invention relates to an oleaginous yeast which is a *Rhodosporidium toruloides* strain deposited in the Spanish Type Culture Collection (CECT) with the following accession number given by the International Depositary Authority: CECT 13234. The ITS region of said strain being SEQ ID NO: 1 or a sequence with a percentage of identity with SEQ ID NO 1, of at least 98%.

This strain was deposited on March 6, 2024, in the Spanish Type Culture Collection, Calle Catedrático Agustin Escardino, 9, CP46980, Paterna (Valencia), Spain, under the Budapest Treaty.

In the present invention strain "CECT13234" and "L3" have the same meaning and both terms can be used interchangeably.

This strain has the ability to:
- Efficiently metabolize different carbon sources, including, without limitation, glucose, xylose, crude glycerin and the sugars present in lignocellulosic biomass hydrolysates.
- Accumulate lipids up to at least 40% of their dry weight when the above carbon sources are used, either separately or in combination.

In the present specification ITS stands for Internal Transcribed Spacer; in a particular embodiment, oleaginous microorganism of the invention comprises and ITS sequence with a percentage of identity with SEQ ID NO 1, of at least 98.1 %, or of at least 98.2%; or of at least 98.3%; or of at least 98.4%; or of at least 98.5%; or of at least 98.6%; or of at least 98.7%; or of at least 98.8%; or of at least 98.9%; or of at least 99%; or of at least 99.1%; or of at least 99.2%; or of at least 99.3%; or of at least 99.4%; or of at least 99.5%; or of at least 99.6%; or of at least 99.7%; or of at least 99.8%; or of at least 99.9%; or 100%.

An additional object of the invention refers to a strain of *Rhodosporidium toruloides* deposited in the Spanish Type Culture Collection under accession number 13234 or mutants derived thereof wherein said mutants have worse, the same or better microbial oil production properties than strain CECT13234.

Mutants derived from this strain refer to strains that are derived from CECT13234, or obtained from CECT13234, and may have mutations compared to *Rhodosporidium toruloides,* preferably, the mutant strain has worse, the same or better microbial oil production properties as the parent strain CECT13234. Preferably, the mutant derived from CECT13234 has at least 80% or more, preferably at least 90% or more, more preferably at least 95% or more or even up to 100% or more than 100% of the microbial-oil production properties compared to the strain CECT13234 under equal conditions.

Microbial oil production properties mean lipid accumulation, speed of growth, and higher range of selection of carbon sources.

The authors of the present invention have developed a novel process for the production of microbial oils, from carbon sources from organic waste such as crude glycerin and/or lignocellulosic waste (lignocellulosic by-products). This process achieves yields comparable to established methods while improving both the quality of the final product and the cost-efficiency of the production process. The process specifically enables the production of a mixture of fatty acid esters, which are suitable as a fuel for diesel-type engines, derived from microbial biomass with a high fatty acid content.

Another object of the invention relates to a process for obtaining microbial oils, which comprise the following steps:
a) production of a microbial biomass with a fatty acid content equal to or greater than 40% by dry weight, by means of the use of the oleaginous microorganism CECT 13234 or mutants derived thereof using organic waste as carbon source;
b) separation of the fatty acid content from the microbial biomass obtained in step a).

In a particular embodiment the fatty acid content is at least 40 % by dry weight, preferably at least 45% by dry weight, even more preferably at least 50 % by dry weight, even more preferably at least 55 % by dry weight, or at least 60 % by dry weight, or at least 65 % by dry weight up to 85% by dry weight, or up to 80 % by dry weight, or up to 75 % by dry weight, or up to 70 % by dry weight In another particular embodiment the fatty acid content in the microbial biomass is comprised between approximately 40% and approximately 70% by dry weight.

In the present invention, the term "fatty acid" and "microbial oils" are synonyms. Fatty acid, comprises fatty acid esters.

In the present invention, the term "microbial biomass" refers to the set of cells of the microorganism once grown in the culture medium.

Herein, the organic waste can be derived from a diverse range of industrial, municipal and/or lignocellulosic by-products derived from agricultural and forestry sources, thus providing a more efficient and sustainable approach to microbial oil production.

In a particular embodiment, organic waste comprises industrial, municipal, agricultural or forestry waste, preferably agricultural or industrial waste, more preferably lignocellulosic by-products and crude glycerin.

Herein the terms "lignocellulosic biomass"; "lignocellulosic by-products" and "lignocellulosic waste" are synonyms.

In a particular embodiment, the organic waste used as carbon source can be any feedstock listed in the Annex IX part A of the Directive (EU) 2018/2001 of the European Parliament and of the Council of 11 December 2018 on the promotion of the use of energy from renewable sources:
a) Algae if cultivated on land in ponds or photobioreactors;
b) Biomass fraction of mixed municipal waste, but not separated household waste subject to recycling targets under point (a) of Article 11(2) of Directive 2008/98/EC;
c) Biowaste as defined in point (4) of Article 3 of Directive 2008/98/EC from private households subject to separate collection as defined in point (11) of Article 3 of that Directive;
d) Biomass fraction of industrial waste not fit for use in the food or feed chain, including material from retail and wholesale and the agro-food and fish and aquaculture industry, and excluding feedstocks listed in part B of this Annex;
e) Straw;
f) Animal manure and sewage sludge;
g) Palm oil mill effluent and empty palm fruit bunches;
h) Tall oil pitch;
i) Crude glycerin;
j) Bagasse;
k) Grape marcs and wine lees;
l) Nut shells;
m) Husks;
n) Cobs cleaned of kernels of corn;
o) Biomass fraction of wastes and residues from forestry and forest-based industries, namely, bark, branches, pre-commercial thinning, leaves, needles, tree tops, saw dust, cutter shavings, black liquor, brown liquor, fiber sludge, lignin and tall oil;
(p) Other non-food cellulosic material;
(q) Other lignocellulosic material except saw logs and veneer logs;

In a particular embodiment, the organic waste, such as tomato waste, zucchini waste, palm shell, almond shell, olive pit, and olive tree pruning is pretreated in order to provide suitable sugars, herein termed lignocellulosic sugars, suitable to be used as carbon source (this is, in the form of hydrolysate).

Preferably the carbon source is selected from the group consisting of one or more of the following organic wastes: crude glycerin, glycerol, lignocellulosic sugars (C5 and/or C6, such us glucose, xylose, galactose, arabinose and mannose).

In a particular embodiment, the organic waste is a combination of crude glycerin and one or more of the following: almond shell hydrolysate and olive pit hydrolysate.

In another particular embodiment the organic waste is selected from one or more of the following: molasses, crude glycerin, lignocellulosic waste, whey, spent coffee grounds, brewery waste, pulp and paper industry effluents, food processing waste, olive mill wastewater, soybean meal, corn steep liquor, wheat straw, rice straw, corn stover, sugarcane bagasse, almond shell, olive pit, potato waste, cassava waste, fruit peels (e.g. orange, banana, apple), vegetable waste, oat hulls and barely straw.

In another particular embodiment crude glycerin is the carbon source, said glycerin is in excess in relation to the nitrogen source present in the same medium, the latter being able to be yeast extract, peptone, corn steep liquor, urea, sodium glutamate or inorganic nitrogen sources such as different ammonium salts, although it is not limited thereto.

In addition to crude glycerin, other carbon sources, such as for example although without limitation, glucose, molasses, sucrose, fructose, starches or fatty acids, can also be present in the culture medium, preferably lignocellulosic sugars, and more preferably lignocellulosic sugars of 5 carbons and/or 6 carbons. Lignocellulosic sugars (C5 and C6) can be obtained from a wide variety of agricultural waste, for example derived from almond shells and/or olive pits. The most common use for this type of waste is energy production through combustion. The process of the invention, on the other hand, valorizes these residues into microbial oil and other high-value bio-based co-products, thus providing a more sustainable and profitable alternative.

According to a particular embodiment of the invention, crude glycerin is used as a carbon source in the fermentation medium, preferably in an amount involving between 40-100%, preferably 90-100% of the total of the carbohydrates used. i.e.,

In the present specification the crude glycerin used is a subproduct of the production of FAME (biodiesel), which is an industrial waste, and it has a purity between approximately 25% or 35% or 50% and 60% or 70% or 80% by weight, preferably between 25% to 70% by weight. Crude glycerin has the advantage of being a cheap and renewable source of carbon.

This glycerin has a very low quality due to the presence of several impurities, which makes it unsuitable for use in the pharmaceutical, cosmetic, food and personal care industries. The oleaginous microorganism and the process of the invention are specially adapted to valorize said residue and use it as substrate for the production of microbial oils.

If the carbon source comprises other carbon sources apart from crude glycerin, and said carbon sources are derived from agricultural waste, preferably lignocellulosic biomass derived from agricultural waste, the process of the invention comprises a stage before step a) of the method of the invention. This stage is needed due to the complex structure of lignocellulosic biomass, which has to be treated to release fermentable sugars and to eliminate specific inhibitory compounds that can hinder microbial growth. This stage is termed Upstream in the present specification.

The lignocellulosic biomass must undergo a series of one or more operations to extract fermentable sugars, which may include physical, chemical, physicochemical and/or biological methods.

Physical methods include operations such as grinding, crushing, cutting, shredding, ball milling, hammer milling, screw pressing, soaking, pelleting, mechanical extrusion, freezing, microwave, and ultrasonication, preferably ball milling or hammer milling.

Chemical methods involve acid hydrolysis (using dilute or concentrated acids, for example HCl, H₂SO₄, and HNO₃, and organic acids, for example oxalic, maleic, and succinic), alkaline or base hydrolysis (using agents like KOH, NaOH, Na₂CO₃, CaO, CaCO₃/Ca(OH)₂, and NH₃), organic solvents, ionic liquids (ILs), and deep eutectic solvents (DESs), preferably acid hydrolysis.

Physicochemical pretreatment or methods include ammonia-based methods (ammonia fiber expansion (AFEX), ammonia recycle percolation (ARP), and soaking aqueous ammonia (SAA)), supercritical fluids (SCF), wet oxidation, steam explosion, and liquid hot water (LHW), preferably steam explosion.

Biological methods involve the utilization of microorganisms (white rot fungi, brown rot fungi, soft rot fungi, lignolytic bacteria, and actinomycetes) and enzymes (peroxidases, such as lignin peroxidase, manganese peroxidase, and versatile peroxidase; laccases; hydrolases, such as cellulases and xylanases; ligases; oxidoreductases; isomerases; transferases; lyases; oxygenases; and dehydrogenases).

These operations of the Upstream stage are well known to a skilled person and the conditions applied are the ones commonly done in the field during routine work.

Additionally, if after the Upstream stage the concentration of inhibitors in the hydrolysate is high enough to negatively affect the fermentation process, a detoxification step will be performed using chemical (treatment with calcium, sodium, ammonium, magnesium, and potassium hydroxides), physical (evaporation, liquid-liquid extraction using organic solvents, liquid-solid extraction with activated charcoal, and membrane-based processes like nanofiltration), and/or biological (microbial detoxification and enzymatic treatment) methods. In a particular embodiment the Upstream stage comprises a detoxification process using Ca(OH)₂, also known as overliming.

A skilled person would know which inhibitors can negatively affect the fermentation process by looking at the state of the art. Preferably, for *Rhodosporidium toruloides,* when in the hydrolysate is present one or more, preferably two or more, more preferably three or more or all of the following inhibitors in a particular concentration:
- formic acid in a concentration of at least 2 g/L,
- acetic acid in a concentration of at least 5 g/L,
- hydroxymethylfurfural (HMF) in a concentration of at least 0.1 g/L, and
- furfural in a concentration of at least 0.1 g/L
a detoxification step is performed to avoid that said inhibitors would negatively affect the fermentation process.

Step a) of the method of the invention is termed Midstream, which involves a fermentation. First, it is required to generate an inoculum of the oleaginous yeast of the invention. It is recommended that the strain grows in medium YPD (Yeast extract Peptone Dextrose) for a suitable time and at an appropriate temperature. Other culture mediums for yeasts known in the field are also suitable.

In a particular embodiment, for the fermentation, the microorganisms are cultured for 1-5 days at a temperature between 18°C and 30°C, preferably between 23°C and 30°C with constant stirring.

The pH of step a) is preferably between 5.8 and 6.2. Different agents can be employed to get a final pH of approximately between 5.8 and 6.2, preferably CaCO₃ and/or K₂CO₃ are used in a concentration of between 1 g/L and 3 g/L, these compounds can be added to the culture medium.

In a particular embodiment the fermentation is carried out in one step (batch mode) or two steps (fed-batch mode), preferably a two-step approach is used, combining batch mode and fed-batch mode, one mode after the other.

In a particular embodiment, fermentation is carried out in two stages: an initial batch phase to maximize microbial growth, followed by a fed-batch phase to maximize intracellular fat accumulation. This combination allows for optimal biomass production in the first stage and enhanced lipid accumulation in the second stage. In the present specification, fat accumulation and microbial oil are synonyms.

In another particular embodiment, the culture medium comprises crude glycerin and non-detoxified olive pit hydrolysate as carbon sources, with a glucose/xylose (G/X) ratio between 10/1 to 25/1, preferably between 12/1 to 22/1; more preferably from 14/1 to 18/1; even more preferably approximately 16/1. This ratio reflects the glucose derived from the crude glycerin relative to the xylose from the olive pit hydrolysate. The amount of glucose and xylose vary depending on the organic waste source, so by establishing a G/X ratio any organic waste source can be used independently on the origin.

Crude glycerin/olive pit hydrolysate proportion may vary significantly depending on the glycerol concentration in the crude glycerin and the sugar concentration in the olive pit hydrolysate. However, maintaining the G/X ratio within the above-mentioned range is most preferred. These desired ratios can be obtained by combining crude glycerin and non-detoxified olive pit hydrolysate in a particular amount.

In another particular embodiment, the culture medium comprises crude glycerin and non-detoxified almond shell hydrolysate as carbon sources, with a glucose/xylose (G/X) ratio between 10/1 to 30/1, preferably between 13/1 to 27/1; more preferably from 16/1 to 23/1; even more preferably approximately 20/1. This ratio reflects the glucose derived from the crude glycerin relative to the xylose from the almond shell hydrolysate. The amount of glucose and xylose vary depending on the organic waste source, so by establishing a G/X ratio any organic waste source can be used independently on the origin.

Crude glycerin/almond shell hydrolysate proportion may vary significantly depending on the glycerol concentration in the crude glycerin and the sugar concentration in the almond shell hydrolysate. However, maintaining the G/X ratio within the above-mentioned range is most preferred. These desired ratios can be obtained by combining crude glycerin and non-detoxified almond shell hydrolysate in a particular amount.

The G/X ratios indicated in the preceding two paragraphs are also applicable when mixing crude glycerin with detoxified olive pit hydrolysate and crude glycerin with detoxified almond shell hydrolysate. The advantage of using detoxified olive pit hydrolysate or detoxified almond shell hydrolysate is that higher yield of microbial oil is obtained.

When the hydrolysates obtained from lignocellulosic waste are used as the sole carbon source, they need to be detoxified before putting them in contact with the microorganisms. However, when said hydrolysates are used in combination with another carbon source such as crude glycerin, and/or glycerol, preferably crude glycerin, the detoxification step becomes optional.

The "sugars" mentioned in the medium composition can originate from various carbon sources, specifically lignocellulosic biomass hydrolysates, such as those derived from olive pits and almond shells or any other source listed in Annex IX part A of the Directive (EU) 2018/2001. If no additional carbon source, such as crude glycerin, is added, these hydrolysates must be detoxified before the fermentation process.

In the experiments disclosed in the present invention, combinations of crude glycerin (as a glucose source) with non-detoxified almond shell hydrolysate (as a xylose source) were tested, with the optimum glucose/xylose (G/X) ratio of 20/1. Additionally, combinations of crude glycerin (as a glucose source) with non-detoxified olive pit hydrolysate (as a xylose source) were evaluated, with the optimum glucose/xylose ratio determined to be 16/1.

In a particular embodiment, the concentration of sugars to start the fermentation of the Midstream step is between 30 g/L and 120 g/L, preferably between 40 g/L and 90 g/L even more preferably between 50 g/L and 70 g/L, preferably the glucose/xylose ratios indicated in the preceding paragraphs are also applicable when determining the concentration of sugars.

The preferred culture mediums of the invention are as follows:
NH₄NO₃ 0.1-10 g/L, CaCl₂·2H₂O 0.1-1.0 g/L, MgSO₄7·H₂O 0.1-1.0 g/L, KH₂PO₄ 0.1-1.0 g/L, corn steep liquor (CSL) 2-10 g/L, crude glycerin and olive pit hydrolysate with a glucose/xylose ratio of 14/1 to 18/1, so the sugar concentration is between: 30-120 g/L, pH: 5.8-6.2, optionally CaCO₃ 0.1-5 g/L or
NH₄NO₃ 0.1-10 g/L, CaCl₂·2H₂O 0.1-1.0 g/L, MgSO₄7·H₂O 0.1-1.0 g/L, KH₂PO₄ 0.1-1.0 g/L, corn steep liquor (CSL) 2-10 g/L, crude glycerin and almond shell hydrolysate with a glucose/xylose ratio of 16/1 to 23/1, so the sugar concentration is between: 30-120 g/L, pH: 5.8-6.2, optionally CaCO₃ 0.1-5 g/L.

The pH, temperature and/or time conditions of the fermentation stage are the usual ones in the field.

In a particular embodiment, the parameters of the Batch mode phase are as follow:
- Culture medium composition, optionally, sterilized at 115-125°C during 15-30 minutes:
   o NH₄NO₃: approximately 0.1 g/L to 10 g/L, or 0.5 g/L to 7.5 g/L
   o CaCl₂·2H₂O: approximately 0.1 g/L to 1.0 g/L, or 0.2 g/L to 0.6 g/L
   o MgSO₄·7H₂O: approximately 0.1 g/L to 1.0 g/L, or 0.2 g/L to 0.6 g/L
   o CaCO₃ and/or K₂CO₃: approximately 0.1 g/L to 5.0 g/L, optional, only when the culture medium comprises lignocellulosic sugars.
   o KH₂PO₄: approximately 0.1 g/L to 1.0 g/L, or 0.5 g/L to 1.0 g/L
   o Corn steep liquor (CSL): approximately 2 g/L to 10 g/L, or 5 g/L to 10 g/L, and
   o Sugars: approximately 30 g/L to 120 g/L, maintaining a suitable G/X ratio as defined above in the preceding paragraphs.
- Inoculum: 1-10% (v/v) of strain L2 in YPD (Yeast Peptone Dextrose) medium for 10 to 30 hours at temperatures between 28°C and 33°C, with agitation at 200-300 rpm.
- pH: 6.0 ± 1, adjusted, for example using H₂SO₄ and NH₄OH solutions.
- C/N ratio: ranges from 9/1 to 60/1.
- Temperature: 28°C to 33°C.
- Dissolved oxygen (dO₂): maintained above 25%.
- Agitation: 200 to 1000 rpm.
- Duration: 12 to 120 hours.
- Foam control: BD-Antiskum 100% (v/v); any other commercially available foam control agent known in the field may also be utilized.

In another particular embodiment, the parameters of the Fed-Batch mode phase are as follow:
- Composition of the feeding:
   o Corn steep liquor (CSL): approximately 1 g/L to 60 g/L, or 1 g/L to 10 g/L, and
   ∘ Crude glycerin and/or lignocellulosic hydrolysates in specific concentrations, determined by their respective compositions.
- Feeding rate: 0.5 to 4.5 mL/min, either constant or variable, based on dissolved oxygen (dO₂) levels.
- pH: 6.0 ± 1, preferably adjusted using H₂SO₄ and NaOH solutions.
- C/N ratio: approximately 400/1 to 600/1, or 50/1 to 600/1, or 100/1 to 600/1, or 200/1 to 600/1, or 300/1 to 600/1, or 400/1 to 600/1, or 500/1 to 600/1, 550/1 to 600/1.
- Temperature: maintained between 28°C and 33°C.
- Dissolved oxygen (dO₂): kept above 5%.
- Duration: conducted over a period of 12 to 120 hours.

Step b) of the method of the invention is termed Downstream. Once the maximum intracellular amount of fatty acid content is reached, the microbial biomass is separated by means of any of the processes normally used for this purpose such as, for example, filtration or centrifugation, following common practices in the field.

Subsequently, the separated microbial biomass is subjected to one or more chemical, physical and/or enzymatic methods to lyse the cells and release the stored microbial oil.

Chemical methods may include, but are not limited to, the use of detergents (ionic and non-ionic), chaotropic agents (such us urea and guanidine hydrochloride), osmotic shock, organic solvents (such as hexane, ethanol, ether, chloroform, or combinations thereof), acid hydrolysis with strong acids (like HCl or H₂SO₄), or alkaline hydrolysis using NaOH or KOH.

In a particular embodiment, the chemical method comprises hydrochloric acid (HCl) at a concentration between 0.1M and 3M, 1 to 5% of dry biomass wt./(dry biomass wt.+HCl solution wt.), a temperature between 40 to 80°C, and a period of time from 1 to 5 hr. So, as will be shown later in the examples, all the fatty acids can be recovered.

Physical methods may comprise high-pressure homogenization (HPH), ball milling, screw press, ultrasonication, microwave treatment, pulsed electric field (PEF) treatment, bead beating, freeze-thaw cycles, osmotic shock, and French press, preferably HPH.

In a particular embodiment, at least one, two, or three passes of HPH are performed at a pressure between 1000 to 2000 bar each, with a wet biomass concentration of 10% wt. to 30% wt. in the homogenizer.

Enzymatic methods may include the use of enzymes such as glucanases, preferably β-glucanases; mannanases, chitinases, proteases, lipases, and cellulases and combinations thereof, preferably a combination of mannanases, β-glucanases and proteases.

In a particular embodiment, a combination of β-glucanases, mannanases, and proteases is applied to microbial biomass at a concentration of 5% to 10% by dry weight, with an enzyme-to-dry biomass ratio of 10% to 25%, a pH of 5 to 6 for β-glucanases, a pH of 8 to 9 for mannanases and proteases, a period of time of 1h to 3h per enzyme, a temperature between 40°C to 65°C, and continuous agitation.

In a particular embodiment, the downstream method comprises a combination of physical and enzymatic methods such as the embodiments disclosed in the three preceding paragraphs.

Once the cells of the microbial biomass have been lysed, a liquid-liquid extraction is performed using organic solvents to concentrate the microbial oil in the organic phase. Examples of suitable solvents include alcohols (such as ethanol, methanol, and isopropanol), hydrocarbons (such as hexane, heptane, cyclohexane, and petroleum ether), esters (such as ethyl acetate, methyl tert-butyl ether, and methyloxolane), other solvents (like chloroform, acetone, and toluene), and solvent mixtures (such as chloroform-methanol and hexane-isopropanol).

In a particular embodiment, two consecutive extractions are carried out with hexane. In a particular embodiment, the first extraction lasts less time than the other, preferably the first one lasts between 1hr and 5 hr and the second extraction between 14hr and 18 hr. The second extraction is performed after the first one.

After the extraction is complete it is necessary to separate the different phases: Organic phase, interphase and aqueous phase, for example by centrifugation, following regular practices in the field. The interesting phase is the organic phase as it comprises the microbial oil.

In a particular embodiment, the organic phase is evaporated to concentrate the microbial oil, this can be done for example by rotary evaporation, reduced-pressure evaporation, flash evaporation, thin-film evaporation, multiple effect evaporation (MEE), simple distillation, or vacuum distillation.

In another particular embodiment, the organic phase is evaporated under vacuum in a rotary evaporator under the following conditions: 1 to 4 hours; cooling temperature: 0°C to 10°C; bath temperature: 50°C to 90°C and/or until most, preferably all, of the organic solvent (preferably hexane) used as the solvent is recovered, and the concentrated microbial oil is obtained.

Each of the terms "comprising," "consisting essentially of," and "consisting of" may be replaced with either of the other two terms. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%; e.g., a weight of "about 100 grams" can include a weight between 90 grams and 110 grams). Use of the term "about" at the beginning of a listing of values modifies each of the values (e.g., "about 1, 2 and 3" refers to "about 1, about 2 and about 3"). When a listing of values is described, the listing includes all intermediate values and all fractional values thereof (e.g., the listing of values "80%, 85% or 90%" includes the intermediate value 86% and the fractional value 86.4%). When a listing of values is followed by the term "or more," the term "or more" applies to each of the values listed (e.g., the listing of "80%, 90%, 95%, or more" or "80%, 90%, 95% or more" or "80%, 90%, or 95% or more" refers to "80% or more, 90% or more, or 95% or more"). When a listing of values is described, the listing includes all ranges between any two of the values listed (e.g., the listing of "80%, 90% or 95%" includes ranges of "80% to 90%", "80% to 95%" and "90% to 95%"). Certain implementations of the technology are set forth in examples below.

### Brief description of the drawings

Fig. 1 Bottles with extracted microbial oils. "4B" and "4" are from L1 strain, while "18" and "20" are from L3 strain.
Fig. 2. Scheme of the process of production of microbial oils. It can be divided in three main steps: Upstream, Midstream and Downstream.

### Examples

### Experiment 1. Isolation and characterization of oleaginous yeast strains

### Isolation and identification of oleaginous yeasts strains

Several soil samples were taken from Andalusian industrial soils, to which a 0.9% NaCl solution was added. The solutions were vortexed. Serial dilutions were prepared and 100 µl was seeded in YPD (Yeast Peptone Dextrose) Petri dishes supplemented with chloramphenicol to prevent bacterial growth. Petri dishes were incubated in an oven at 30°C for 72 hours.

For each sample, large colonies were selected for microscopic observation. After microscopic observations, the selected yeasts were identified by molecular biology techniques. For this, genomic DNA extractions were performed, the ITS region of each strain was amplified and sequenced. 4 strains were finally chosen: 3 strains from *R. toruloides:* L1, L2, L3 and 1 strain from *Yarrowia lipolytica:* L4

### Characterization of oleaginous yeasts strains in various culture media

The characterization of each oleaginous yeast was carried out by performing growth assays for the evaluation of both cell biomass and total fat production. Five culture media with different carbon sources were used for this purpose. The compositions of these 5 cultures mediums are as follows:
Medium GX
   NH₄NO₃ 0.7 g/L, CaCl₂·2H₂O 0.4 g/L, MgSO₄·7H₂O 0.4 g/L, KH₂PO₄ 0.75 g/L, corn steep liquor 9.6 g/L, glucose 70 g/L, xylose 40 g/L. Final pH: 6.0.
Medium Gli "low glycerol"
   NH₄NO₃ 0.7 g/L, CaCl₂·2H₂O 0.4 g/L, MgSO₄·7H₂O 0.4 g/L, KH₂PO₄ 0.75 g/L, corn steep liquor 9.6 g/L, crude glycerin 110 g/L (37.6% wt. of glycerol). Final pH: 6.0.
Medium Gli "high glycerol"
   NH₄NO₃ 0.35 g/L, CaCl₂·2H₂O 0.2 g/L, MgSO₄·7H₂O 0.2 g/L, KH₂PO₄ 0.38 g/L, corn steep liquor 9.6 g/L, crude glycerin 55 g/L (64.1% wt. of glycerol). Final pH: 6.0.
Medium "Olive tree pruning"
   NH₄NO₃ 0.7 g/L, CaCl₂·2H₂O 0.4 g/L, MgSO₄·7H₂O 0.4 g/L, KH₂PO₄ 0.75 g/L, corn steep liquor 9.6 g/L, olive tree pruning hydrolysate 110 g/L, glucose 20 g/L, xylose 11 g/L. Final pH: 6.0.
Medium "Olive pit"
   NH₄NO₃ 0.7 g/L, CaCl₂·2H₂O 0.4 g/L, MgSO₄·7H₂O 0.4 g/L, KH₂PO₄ 0.75 g/L, corn steep liquor 9.6 g/L, olive pit hydrolysate 55 g/L, glucose 20 g/L, CaCO₃ 1 g/L. Final pH: 6.0.

To perform the growth assays, we have relied on the literature, reviewing scientific articles and patents in order to use a method and a culture medium suitable for *R*. *toruloides.* The tests were carried out as follows:
1-Pre-inoculum preparation: each strain was inoculated from a plate into 10 mL of liquid YPD culture medium and incubated for 24 hours at 30°C with shaking at 250 rpm.

2-Fermentation: fermentations were carried out in 500 mL flasks with 100 mL of the respective culture medium being tested. Corn steep liquor (CSL) (EC number: 266-113-4; CAS number: 66071-94-1) was pre-sterilized (prepared at 50% v/v, pH 6.0) and added at the start of the fermentation, achieving a final CSL concentration of 9.6 g/L. Flasks were inoculated with an initial cell density of OD₆₀₀ = 0.1 and incubated for 96 hours at 30°C with agitation at 250 rpm.

3-Cell biomass processing: At the end of the fermentation, the cultures were examined microscopically. A 90 mL sample from each culture was centrifuged for 10 minutes at 10,000 rpm. The resulting pellets were washed with 10 mL of a 50% ethanol solution, followed by another centrifugation to remove excess liquid. The wet pellets were then dried in an oven at 60°C for 24 hours to determine the dry weight and assess the biomass produced during fermentation.

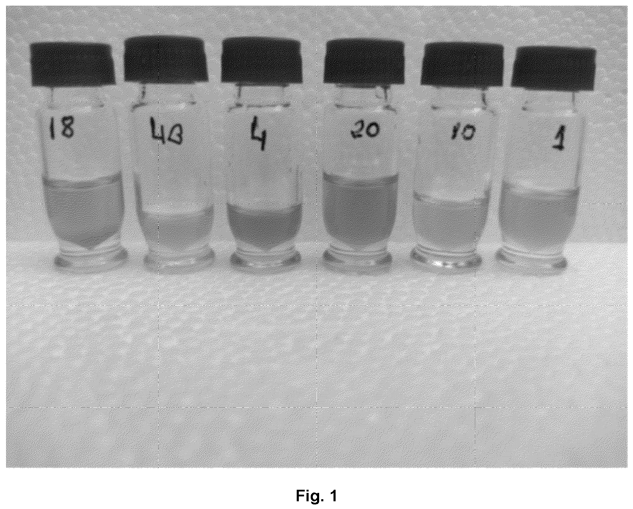

4-Total fat extraction: The total lipid content from the oleaginous yeasts was extracted using the Soxhlet method under heated conditions. A 1g sample was digested in 40 mL of 3N HCl for 1 hour. The hydrolysate was filtered through paper, washed three times with water (250 mL in total), and the filter was dried in an oven at 100°C for 10 minutes. The dried filter was then placed in a 100 mL Soxhlet extraction cartridge and subjected to hexane reflux for 4 hours, siphoning every 10-12 minutes. The resultant extract was concentrated by rotary evaporation, and the remaining residue was weighed to calculate the total fat percentage relative to the initial dry weight (Fig. 1).

Table 1 provides a detailed summary of the biomass and lipid content produced by the four oleaginous yeast strains cultivated in various media. This data highlights the differences in lipid accumulation among the yeast strains under different culture conditions.

It can be observed that strain L3 showed the highest accumulation of total fat (% wt.) among the strains tested.

**Table 1: Comparative biomass and lipid content of four oleaginous yeast strains cultivated in different media.**

| **Culture medium** | **Yeast strain** | **Biomass (g)** | **Biomass (g/L)** | **Fat (g)** | **Fat (g/L)** | **Fat (% wt.)*** |
|---|---|---|---|---|---|---|
| GX | L1 | 1.08 | 11.99 | 0.44 | 4.89 | 40.78 |
| | L2 | 1.20 | 13.27 | 0.42 | 4.71 | 35.49 |
| | L3 | 1.19 | 13.18 | 0.54 | 6.01 | 45.60 |
| | L4 | 0.48 | 5.29 | 0.10 | 1.15 | 21.74 |
| Gli "low glycerol" | L1 | 0,.87 | 9.67 | 0.32 | 3.59 | 37.13 |
| | L2 | 1.13 | 12.58 | 0.42 | 4.66 | 37.04 |
| | L3 | 1.17 | 12.99 | 0.55 | 6.13 | 47.19 |
| | L4 | 0.62 | 6.93 | 0.12 | 1.37 | 19.77 |
| Gli "high glycerol" | L1 | 0.70 | 7.76 | 0.19 | 2.07 | 26.68 |
| | L2 | 1.05 | 11.69 | 0.32 | 3.50 | 29.94 |
| | L3 | 1.04 | 11.53 | 0.38 | 4.23 | 36.69 |
| | L4 | 0.70 | 7.78 | 0.08 | 0.86 | 11.05 |
| Olive tree pruning | L1 | 0.82 | 9.12 | 0.22 | 2.43 | 26.64 |
| | L2 | 0.86 | 9.50 | 0.23 | 2.61 | 27.47 |
| | L3 | 0.86 | 9.56 | 0.20 | 2.24 | 23.43 |
| | L4 | 0.51 | 5.71 | 0.08 | 0.93 | 16.29 |
| Olive pit** | L2 | 1.94 | 21.56 | 1.08 | 12.01 | 55.71 |
| | L3 | 1.80 | 20.02 | 1.05 | 11.71 | 58.49 |

| | | | | | | |
|---|---|---|---|---|---|---|
| *% of dry weight. **In this culture medium, L1 and L4 were not tested. | | | | | | |

### Experiment 2. Growth and lipid production by oleaginous yeasts using crude glycerin and non-detoxified hydrolysates

The sugar concentration prior to the Midstream stage was set at 60 g/L, achieved through the combination of two different carbon sources:
- Glucose, supplied in the form of crude glycerin as used in the medium of Experiment 1 Gli "high glycerol", containing 64.1% by weight of glycerol.
- Xylose, introduced into the culture medium through non-detoxified hydrolysates obtained from olive pits or almond shells.

The preparation of the culture media was conducted by adjusting different glucose/xylose ratios. Table 2 provides the composition of the carbon sources, the specific glucose/xylose ratios, and the corresponding biomass production of L2 and L3 strains for each of the ten cultures media formulations:

**Table 2: Composition of the carbon sources and biomass production of L2 and L3 strains for the different culture media formulations.**

| **Culture medium** | **Carbon Source (CS)** | **CS (g/L)** | **G/X Ratio** | **L2 strain (g/L)** | **L3 strain (g/L)** |
|---|---|---|---|---|---|
| OLIVE A | Glucose (Crude glycerin) | 38.18 | 1.75/1 | 0.7 | 1.8 |
| | Xylose (Olive pit hydrolysate) | 21.82 | | | |
| OLIVE B | Glucose (Crude glycerin) | 48.00 | 4/1 | 11.1 | 12.8 |
| | Xylose (Olive pit hydrolysate) | 12.00 | | | |
| OLIVE C | Glucose (Crude glycerin) | 53.33 | 8/1 | 14.3 | 14.9 |
| | Xylose (Olive pit hydrolysate) | 6.67 | | | |
| OLIVE D | Glucose (Crude glycerin) | 56.49 | 16.1/1 | 16.5 | 16.1 |
| | Xylose (Olive pit hydrolysate) | 3.51 | | | |
| OLIVE E | Glucose (Crude glycerin) | 57.20 | 20.4/1 | 16.1 | 15.2 |
| | Xylose (Olive pit hydrolysate) | 2.80 | | | |
| ALMOND A | Glucose (Crude glycerin) | 38.18 | 1.751 | 0.7 | 1.5 |
| | Xylose (Almond shell hydrolysate) | 21.82 | | | |
| ALMOND B | Glucose (Crude glycerin) | 48.00 | 4/1 | NA* | 11.6 |
| | Xylose (Almond shell hydrolysate) | 12.00 | | | |
| ALMOND C | Glucose (Crude glycerin) | 53.33 | 8/1 | 13.8 | 13.0 |
| | Xylose (Almond shell hydrolysate) | 6.67 | | | |
| ALMOND D | Glucose (Crude glycerin) | 56.49 | 16.1/1 | 14.2 | 15.9 |
| | Xylose (Almond shell hydrolysate) | 3.51 | | | |
| ALMOND E | Glucose (Crude glycerin) | 57.20 | 20.4/1 | 16.0 | 15.7 |
| | Xylose (Almond shell hydrolysate) | 2.80 | | | |

| | | | | | |
|---|---|---|---|---|---|
| *NA: Not available | | | | | |

For each culture medium, the sum of the values of the CS columns gives an amount of 60 g/L, which is the sugar concentration. The G/X ratio is the glucose/xylose ratio, for each culture medium this value is obtained by dividing the glucose of the crude glycerin between the xylose of the hydrolysates.

In addition, each culture medium was prepared by adding various salts and a nitrogen source (corn steep liquor), as indicated below. The final pH was adjusted to 6.0 ± 0.1.

Final composition of each culture medium:
NH₄NO₃ 0.7 g/L, CaCl₂·2H₂O 0.4 g/L, MgSO₄·7H₂O 0.4 g/L, KH₂PO₄ 0.75 g/L, corn steep liquor 9.6 g/L, sugars 60 g/L. Final pH: 6.0.

Assays were performed in 100 mL flasks with 20 mL of each medium (designated as OLIVE A, B, C, D, or E; ALMOND A, B, C, D, or E). Each flask was inoculated at 1% with a pre-culture of strains L2 or L3 previously grown in YPD medium and incubated for 48h to 72h at 30°C, under agitation at 250 rpm. Biomass production was measured after centrifugation, drying, and weighing of the dry pellets.

The best results were observed for the following cultures media:
- OLIVE D: Olive pit hydrolysate + Crude glycerin, glucose/xylose ratio of 16.1/1.
- ALMOND E: Almond shell hydrolysate + Crude glycerin, glucose/xylose ratio of 20.4/1.

Due to the presence of growth inhibitors (mainly acetic acid) in "OLIVE" and "ALMOND" cultures media, a reduction in initial acidity using an industrial deacidifying compound was considered. Growth assays were conducted by adding calcium carbonate (CaCO₃) at 1g/L or 3 g/L, or potassium carbonate (K₂CO₃) at 1g/L to the "OLIVE D" and "ALMOND E" cultures media. In order to adjust the pH to 6.0 ± 0.1 any other deacidifying compound can be used while the final pH before fermentation is approximately 6.

The addition of deacidifying compounds improved cell growth, with the best results obtained at CaCO₃ 3 g/, leading to an increase in growth of approximately 10-12% compared to cultures without deacidifying compounds.

These experiments have enabled the determination of the optimal composition of the culture media for conducting fermentations with L2 and L3 strains:
"OLIVE" culture medium:
   NH₄NO₃ 0.7 g/L, CaCl₂·2H₂O 0.4 g/L, MgSO₄7·H₂O 0.4 g/L, KH₂PO₄ 0.75 g/L, corn steep liquor 9.6 g/L, crude glycerin and olive pit hydrolysate with a glucose/xylose ratio of 16.1/1, CaCO₃ 3 g/L. Final pH: 6.0.
   "ALMOND" culture medium:
      NH₄NO₃ 0.7 g/L, CaCl₂·2H₂O 0.4 g/L, MgSO₄·7H₂O 0.4 g/L, KH₂PO₄ 0.75 g/L, corn steep liquor 9.6 g/L, crude glycerin and almond shell hydrolysate with a glucose/xylose ratio of 20.4/1, CaCO₃ 3 g/L. Final pH: 6.0.

The protocol to determine total lipid accumulation was as follows:
1-Pre-inoculum preparation: each strain was inoculated from a plate into 20 mL of YPD liquid culture medium and incubated for 24 hours at 30°C with shaking at 250 rpm.

2-Fermentation: fermentations were carried out in 1L flasks with 200 mL of the respective culture medium being tested. Corn steep liquor (CSL) was pre-sterilized (prepared at 50% v/v, pH 6.0) and added at the start of the fermentation, achieving a final CSL concentration of 9.6 g/L. Flasks were inoculated with an initial cell density of OD₆₀₀ = 0.1 and incubated for 120 hours at 30°C with agitation at 250 rpm.

3-Cell biomass processing: at the end of the fermentation, the cultures were examined under the microscope and 150 mL of each culture was centrifuged for 10 minutes at 10,000 rpm. The resulting pellets were washed with 15 mL of 50% ethanol solution and centrifuged again to remove excess liquid. The wet pellets were dried in an oven at 65°C for 48 hours to isolate the dry pellet, which was then used to evaluate the cell biomass produced during fermentation.

4-Total fat extraction: the total lipid content from the oleaginous yeasts was extracted using the Soxhlet method under heated conditions. A 2.5 g of sample was digested in 100 mL of 3N HCl for one hour. The hydrolysate was filtered through paper, and after three washes with water (250 mL in total), the filter was dried in an oven at 100°C for 10 minutes. The dried filter was then placed in a 100 mL Soxhlet extraction cartridge and subjected to hexane reflux for 4 hours, siphoning every 10-12 minutes. The resultant extract was concentrated by rotary evaporation, and the remaining residue was weighed to determine the total fat percentage relative to the initial dry weight.

The production of cell biomass and total fat in the OLIVE and ALMOND culture media is outlined in Table 3.

**Table 3: Biomass and total fat production of L2 and L3 strains in optimized OLIVE and ALMOND culture media.**

| **Culture medium** | **Yeast strain** | **Biomass (g)** | **Biomass (g/L)** | **Fat (g)** | **Fat (g/L)** | **Fat (% wt.)** |
|---|---|---|---|---|---|---|
| OLIVE | L2 | 2.85 | 19.00 | 1.30 | 8.65 | 45.53 |
| | L3 | 2.91 | 19.40 | 1.35 | 9.00 | 46.39 |
| ALMOND | L2 | 2.96 | 19.70 | 1.40 | 9.32 | 47.31 |
| | L3 | 2.94 | 19.60 | 1.43 | 9.53 | 48.62 |

Therefore, the best results obtained were:
- 46.4% by weight, when using crude glycerin and non-detoxified olive pit hydrolysate with a glucose/xylose ratio of 16/1 as carbon source.
- 48.6% weight, when using crude glycerin and non-detoxified almond shell hydrolysate with a glucose/xylose ratio of 20/1 as carbon source.

### Experiment 3. Growth and lipid production by oleaginous yeasts using crude glycerin and/or detoxified hydrolysates

A series of assays were performed using the strains L2, the strain of the invention CECT 13234 (L3), and CECT 1137 (a publicly available strain of *Rhodosporidium toruloides* from the public collection) to demonstrate that strains L2 and L3 produce significantly more microbial oil than any other strain of the same species.

These tests were conducted employing various raw materials as carbon sources:
- Glucose, supplied in the form of crude glycerin as used in the medium of Experiment 1 Gli "low glycerol", containing 37.6% by weight of glycerol.
- Xylose, introduced into the culture medium through detoxified hydrolysates obtained from olive pits or almond shells.

The compositions of the culture mediums are as follows:
Medium "Crude glycerin"
   NH₄NO₃ 0.7 g/L, CaCl₂·2H₂O 0.4 g/L, MgSO₄·7H₂O 0.4 g/L, KH₂PO₄ 0.75 g/L, corn steep liquor 9.6 g/L, crude glycerin 110 g/L. Final pH: 6.0.
Medium "Olive pit"
   NH₄NO₃ 0.7 g/L, CaCl₂·2H₂O 0.4 g/L, MgSO₄·7H₂O 0.4 g/L, KH₂PO₄ 0.75 g/L, corn steep liquor 9.6 g/L, olive pit hydrolysate 434.09 g/L, CaCO₃ 3 g/L. Final pH: 6.0.
Medium "Olive + Glycerin"
   NH₄NO₃ 0.7 g/L, CaCl₂·2H₂O 0.4 g/L, MgSO₄·7H₂O 0.4 g/L, KH₂PO₄ 0.75 g/L, corn steep liquor 9.6 g/L, crude glycerin and olive pit hydrolysate with a glucose/xylose ratio of 16.1/1, CaCO₃ 3 g/L. Final pH: 6.0.
Medium "Almond shell"
   NH₄NO₃ 0.7 g/L, CaCl₂·2H₂O 0.4 g/L, MgSO₄·7H₂O 0.4 g/L, KH₂PO₄ 0.75 g/L, corn steep liquor 9.6 g/L, almond shell hydrolysate 519.12 g/L, CaCO₃ 3 g/L. Final pH: 6.0.
Medium "Almond +Glycerin"
   NH₄NO₃ 0.7 g/L, CaCl₂·2H₂O 0.4 g/L, MgSO₄·7H₂O 0.4 g/L, KH₂PO₄ 0.75 g/L, corn steep liquor 9.6 g/L, crude glycerin and almond shell hydrolysate with a glucose/xylose ratio of 20.4/1, CaCO₃ 3 g/L. Final pH: 6.0.

For each culture medium, the sugar concentration was 60 g/L.

All assays were conducted in duplicate following the protocol outlined below:
1-Pre-inoculum preparation: each strain was inoculated from a plate into 20 mL of liquid YPD culture medium and incubated for 24 hours at 30°C with shaking at 250 rpm.
2-Fermentation: fermentations were carried out in 1 L flasks with 200 mL of the respective culture medium being tested. Corn steep liquor (CSL) was pre-sterilized (prepared at 50% v/v, pH 6.0) and added at the start of the fermentation, achieving a final CSL concentration of 9.6 g/L. Flasks were inoculated with an initial cell density of OD₆₀₀ = 0.1 and incubated for 120 hours at 30°C with agitation at 250 rpm.
3-Cell biomass processing: at the end of the fermentation, the cultures were examined microscopically. A 150 mL sample from each culture were centrifuged for 10 minutes at 10,000 rpm. The resulting pellets were washed with 15 mL of a 50% ethanol solution, followed by another centrifugation to remove excess liquid. The wet pellets were then dried in an oven at 65°C for 48 hours to determine the dry weight and assess the biomass produced during fermentation.
4-Total fat extraction: The total lipid content from the oleaginous yeasts was extracted using the Soxhlet method under heated conditions. A 2.5 g sample was digested in 100 mL of 3N HCl for 1 hour. The hydrolysate was filtered through paper, washed three times with water (250 mL in total), and the filter was dried in an oven at 100°C for 10 minutes.
The dried filter was then placed in a 100 mL Soxhlet extraction cartridge and subjected to hexane reflux for 4 hours, siphoning every 10-12 minutes. The resultant extract was concentrated by rotary evaporation, and the remaining residue was weighed to calculate the total fat percentage relative to the initial dry weight.

Table 4 presents the results of cell biomass production and total fat accumulation in the tested culture media.

**Table 4. Biomass and lipid production by CECT1137, L2, and L3 strains in various culture media**

| **Culture medium** | **Yeast strain** | **Biomass (g)** | **Biomass (g/L)** | **Fat (g)** | **Fat (g/L)** | **Fat (% wt.)** |
|---|---|---|---|---|---|---|
| Crude glycerin | CECT 1137 | 0.94 | 6.28 | 0.19 | 1.26 | 19.99 |
| | L2 | 1.89 | 12.58 | 0.61 | 4.08 | 32.43 |
| | L3 | 1.95 | 12.99 | 0.78 | 5.18 | 39.88 |
| Olive pit | CECT 1137 | 1.94 | 12.92 | 0.53 | 3.54 | 27.40 |
| | L2 | 2.37 | 15.78 | 1.04 | 6.91 | 43.79 |
| | L3 | 2.33 | 15.52 | 1.03 | 6.88 | 44.33 |
| Olive + Glycerin | CECT 1137 | 1.77 | 11.81 | 0.38 | 2.52 | 21.34 |
| | L2 | 2.72 | 18.13 | 1.33 | 8.87 | 48.92 |
| | L3 | 2.48 | 16.52 | 1.40 | 9.30 | 56.30 |
| Almond shell | CECT 1137 | 2.13 | 14.17 | 0.78 | 5.23 | 36.91 |
| | L2 | 2.25 | 14.98 | 0.99 | 6.59 | 43.99 |
| | L3 | 2.25 | 15.01 | 0.99 | 6.60 | 43.97 |
| Almond + Glycerin | CECT 1137 | 1.54 | 10.27 | 0.33 | 2.22 | 21.62 |
| | L2 | 2.62 | 17.49 | 1.27 | 8.47 | 48.43 |
| | L3 | 2.56 | 17.04 | 1.38 | 9.22 | 54.11 |

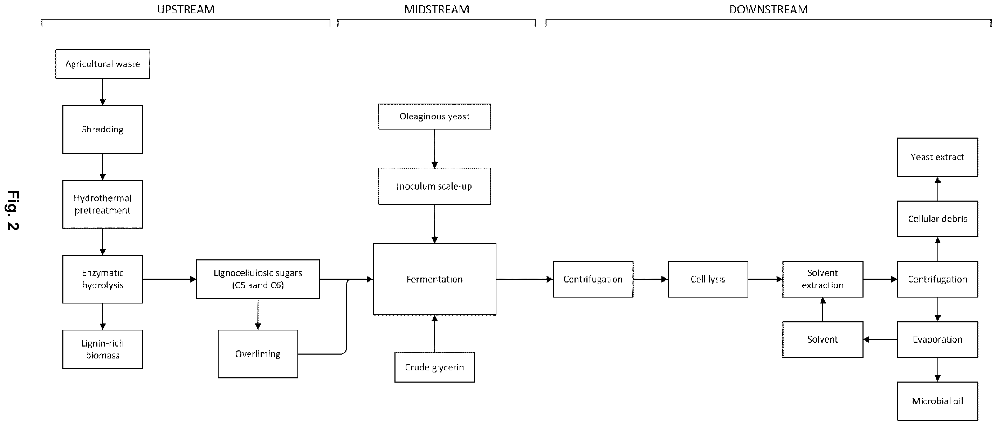

The process of the invention can be split into 3 main steps Upstream, Midstream and Downstream Fig. 2

### Upstream

The Upstream step includes essential operations to prepare low-cost, waste raw materials, preferably lignocellulosic biomass, making it suitable as carbon sources for fermentation. While crude glycerin can be directly metabolized by the microorganism without prior pretreatment, agricultural residues require a series of processes to release lignocellulosic sugars (C5 and C6). These processes involve shredding, hydrothermal pretreatment, and enzymatic hydrolysis.

Shredding reduces the biomass into small, uniform particles, improving surface area and accessibility for subsequent hydrothermal pretreatment. This step typically uses equipment like hammer mills, ball mills, crushers, or shredders to achieve the desired particle size reduction.

Once shredded, the biomass is subjected to a hydrothermal pretreatment, for example a steam explosion (SE) under acidic conditions. This method involves treating the crushed biomass with saturated steam at a temperature of 185-215°C and an addition of 0-40 mg of H₂SO₄ per gram of biomass. The pretreatment is conducted for 5-10 minutes, depending on the specific type of agricultural residue, to facilitate the hydrolysis of hemicellulose and enhance cellulose saccharification.

After the SE process, the pretreated material is collected in a cyclone, cooled to approximately 40°C, and subsequently filtered to separate two distinct fractions: (i) the insoluble solids fraction and (ii) the liquid filtrate or hydrolysate. The insoluble solids fraction mainly contains cellulose, lignin, and ash, while the liquid fraction contains solubilized hemicellulosic sugars (C5 and C6) and degradation byproducts such as furans, short-chain carboxylic acids, and phenolic derivatives.

In lieu of the SE process, we performed the hydrothermal pretreatment using a laboratory autoclave, a widely available equipment in research facilities. While an autoclave does not exactly replicate the SE process, particularly lacking the explosive decompression, it does allow for the simulation of similar hydrothermal conditions on a much smaller scale. This enables preliminary tests to be conducted with minimal sample quantities, which is particularly advantageous in preliminary tests. The conditions employed during these tests were as follows: autoclave at 125°C for 30 minutes, with 2% H₂SO₄ and a solid-to-liquid (S/L) ratio of 50%, conducted across two contacts. Table 5 summarizes the gravimetric recovery and mass of pretreated almond shells after the hydrothermal pretreatment described above.

**Table 5. Material balance**

| **Parameter** | **Value** |
|---|---|
| Initial mass of raw material (almond shells) (g) | 100.00 |
| Gravimetric recovery (1^{st} contact) (% wt.) | 79.13 |
| Mass of pretreated almond shells (1^{st} contact) (g) | 79.13 |
| Gravimetric recovery (2^{st} contact) (%wt.) | 77.60 |
| Mass of pretreated almond shells (2^{nd} contact) (g) | 61.40 |

Two contacts mean that a 1^{st} contact was made under the conditions shown: 125 °C, 30 minutes, 2% H₂SO₄, 50% S/L ratio and, once this initial step was completed, the resulting hydrolysate was returned to the autoclave under the same conditions in order to increase sugar extraction, as shown in Tables 6, 7 and 8.

Gravimetric recovery provides insight into the degree of solubilization of the material resulting from the hydrothermal process and the proportion of solubilized sugars. These components are measured via high-performance liquid chromatography (HPLC), following regular practices in the field.

### 1^{st} contact

| | |
|---|---|
| S/L ratio | 50% |
| Acid volume (mL) | 100 |
| Raw material mass (g) | 50 |

**Table 6. Composition of solubilized components following 1^{st} contact hydrolysis**

| **Component** | **Concentration (g/L)** | **Mass (g)** | **Mass (g/100g raw material)** |
|---|---|---|---|
| Glucose | 1.749 | 0.17 | 0.35 |
| Xylose | 59.980 | 6.00 | 12.00 |
| Galactose | 6.791 | 0.68 | 1.36 |
| Arabinose | 4.135 | 0.41 | 0.83 |
| Mannose | 0.241 | 0.02 | 0.05 |
| Formic acid | 0.690 | 0.07 | 0.14 |
| Acetic acid | 17.270 | 1.73 | 3.45 |
| Hydroxymethylfurfural (HMF) | 0.017 | 0.00 | 0.00 |
| Furfural | 0.187 | 0.02 | 0.04 |

### 2^{st} contact

| | |
|---|---|
| S/L ratio | 50% |
| Acid volume (mL) | 100 |
| Raw material mass (g) | 50 |

**Table 7. Composition of solubilized components following 2^{nd} contact hydrolysis**

| **Component** | **Concentration (g/L)** | **Mass (g)** | **Mass (g/100g raw material)** |
|---|---|---|---|
| Glucose | 1.450 | 0.15 | 0.23 |
| Xylose | 70.988 | 7.10 | 11.23 |
| Galactose | 5.119 | 0.51 | 0.81 |
| Arabinose | 1.612 | 0.16 | 0.26 |
| Mannose | 0.192 | 0.02 | 0.03 |
| Formic acid | 0.780 | 0.08 | 0.12 |
| Acetic acid | 18.860 | 1.89 | 2.98 |
| Hydroxymethylfurfural (HMF) | 0.000 | 0.00 | 0.00 |
| Furfural | 0.827 | 0.08 | 0.13 |

**Table 8. Summary (g/100g raw material)**

| **Component** | **Initial mass (g/100g r.m.)** | **1^{st} contact (g/100g r.m.)** | **2^{nd} contact (g/100g r.m.)** |
|---|---|---|---|
| Glucose | 21.34 | 0.35 | 0.23 |
| Xylose | 29.82 | 12.00 | 11.23 |
| Galactose | 1.21 | 1.36 | 0.81 |
| Arabinose | 1.06 | 0.83 | 0.26 |
| Mannose | 0.00 | 0.05 | 0.03 |
| Formic acid | - | 0.14 | 0.12 |
| Acetic acid | - | 3.45 | 2.98 |
| Hydroxymethylfurfural (HMF) | - | 0.00 | 0.00 |
| Furfural | - | 0.04 | 0.13 |
| **Mass of pretreated almond shells (g)** | **-** | 79.13 | 61.40 |
| **TOTAL BALANCE (g)** | | | 95.42 |

The recovery of xylose in the process is 40% after the first contact and increases to 78% after the second contact. The initial 40% is calculated by dividing the amount of xylose obtained in the first contact by the original xylose content present in the almond shells prior to the hydrothermal pretreatment. The 78% is obtained by summing the xylose recovered from both the first and second contacts, again dividing by the initial xylose content. After the second contact, 61% of the solid material remains available, making it suitable for different applications.

These promising results, obtained using a laboratory autoclave, are likely to be further enhanced when using actual SE equipment due to the additional mechanical effects of the rapid decompression.

The hydrolysates resulting from the hydrothermal pretreatment usually contain degradation products that are known to impede microbial metabolism, thereby hindering cell growth. However, when crude glycerin and lignocellulosic hydrolysates are jointly used as carbon sources during the subsequent fermentation stage, the strain employed in this process demonstrates robust growth, negating the need for inhibitor removal. In contrast, when using only lignocellulosic hydrolysates, a detoxification step becomes essential to reduce the concentration of inhibitory compounds and enhanced microbial viability.

Detoxification is achieved through the overliming process, where calcium hydroxide is added to the hydrolysate at 50°C until the pH reaches 10. Subsequently, concentrated sulfuric acid is introduced to bring the pH down to 2.5. The resulting precipitate, containing the majority of the inhibitors, is separated through centrifugation at 3,500 rpm for 10 minutes using regular procedures. This method effectively reduces the concentration of hydroxymethylfurfural (HMF) and furfural, two of the most toxic inhibitors for *Rhodosporidium toruloides,* even at levels below 1 g/L. Additionally, it also decreases the concentrations of formic acid and acetic acid. The efficiency of the overliming process in reducing inhibitor concentrations is detailed in Tables 9 and 10.

**Table 9: Detoxification efficiency of the overliming treatment on olive pit hydrolysate**

| **Component** | **Concentration before overliming (g/L)** | **Concentration after overliming (g/L)** | **Reduction (%)** |
|---|---|---|---|
| Formic acid | 1.34 | 0.50 | 62.69 |
| Acetic acid | 24.17 | 20.56 | 14.94 |
| Ethanol | 0.31 | 0.33 | -6.45 |
| Hydroxymethylfurfural (HMF) | 0.86 | 0.22 | 74.42 |
| Furfural | 1.11 | 0.21 | 81.08 |

**Table 10: Detoxification efficiency of the overliming treatment on almond shell hydrolysate**

| **Component** | **Concentration before overliming (g/L)** | **Concentration after overliming (g/L)** | **Reduction (%)** |
|---|---|---|---|
| Formic acid | 0.64 | 0.50 | 21.88 |
| Acetic acid | 14.17 | 11.97 | 15.53 |
| Ethanol | 0.51 | 0.57 | -11.76 |
| Hydroxymethylfurfural (HMF) | 0.77 | 0.28 | 63.64 |
| Furfural | 1.04 | 0.11 | 89.42 |

The enzymatic hydrolysis-is performed on the insoluble solids fraction obtained after the SE using commercial enzymes, which may include one or more of the following: endoglucanases (EG), exoglucanases or cellobiohydrolases (CBH), and β-glucosidases (BG). Enzyme loading ranges from approximately 2.5 to 20 filter paper units (FPU) per gram of cellulose, depending on the specific enzyme blend. The process is carried out at a solid substrate concentration of 2% to 5% (w/v) under controlled pH conditions, maintained between 4.8 and 5.0. The temperature of the reaction is regulated between 45°C and 50°C, and the hydrolysis is allowed to proceed for a duration of 24 to 72 hours, depending on the nature of the lignocellulosic material. The objective of this process is to hydrolyze cellulose into glucose (C6) through the application of commercial enzymes, while concurrently separating lignin, which is inhibitory to microbial fermentation. Upon completion of this stage, two distinct fractions are produced: (i) a liquid fraction substantially enriched with glucose, and (ii) a solid fraction predominantly composed of lignin.

The lignin-rich solid fraction is subsequently separated from the process stream and subjected to further valorization, as it has significant potential for utilization in the fabrication of bio-based materials, advanced adhesives, and high-performance carbon fibers.

The integration of hydrothermal pretreatment and enzymatic hydrolysis facilitates the extraction of almost all fermentable sugars contained within the lignocellulosic biomass, significantly enhancing process efficiency. This approach enables the recovery of hemicellulose-derived sugars (C5 and C6) and cellulose-derived glucose (C6), which are subsequently utilized as carbon sources during the Midstream stage.

### Midstream

In this stage, fermentable sugars derived from industrial and/or agricultural residues are biotransformed into microbial oils.

The fermentation process was carried out in two distinct stages: an initial batch mode phase, aimed at maximizing microbial growth, followed by a fed-batch mode phase to enhance intracellular fat accumulation.

Batch Phase mode parameters:
- Inoculum: 1-3% (v/v) of strain L2 or L3 grown in YPD (Yeast Peptone Dextrose) medium for 12 to 24 hours at temperatures between 28°C and 33°C, with agitation at 200-300 rpm.
- Culture medium composition:
   o NH₄NO₃: 0.7 g/L, 3 g/L or 7 g/L
   o CaCl₂: 0.4 g/L
   o MgSO₄: 0.4 g/L
   o CaCO₃ and/or K₂CO₃: 1 g/L or 3 g/L, optional, only when the culture medium comprises lignocellulosic sugars.
   o KH₂PO₄: 0.75 g/L
   o Corn steep liquor (CSL): 2.9 g/L, 5 g/L or 9.6 g/L
   o Sugars: 60 g/L.
- pH: 6 ± 0.5, adjusted using H₂SO₄ (1.5M) and NH₄OH (25% v/v) solutions.
- C/N ratio: 9.4/1, 18/1, 38/1 or 53/1.
- Temperature: 28°C to 33°C.
- Dissolved oxygen (dO₂): maintained above 30%.
- Agitation: ranging from 300 to 1000 rpm.
- Duration: 24 to 48 hours.
- Foam control: BD-Antiskum 100% (v/v); any other commercially available foam control agent known in the field may also be utilized.

Fed-Batch Phase mode parameters:
- Composition of the feeding:
   o Corn steep liquor (CSL): 0 g/L, 5.3 g/L or 53.2 g/L, and
   o Crude glycerin and/or lignocellulosic hydrolysates in specific concentrations, determined by their respective compositions.
- Feeding rate: 0.53 to 4.33 mL/min, either constant or variable, based on dissolved oxygen (dO₂) levels.
- pH: 6 ± 0.5, controlled by the addition of H₂SO₄ (1.5M) and NaOH (3M) solutions.
- C/N ratio: 57/1 or 575/1.
- Temperature: maintained between 28°C and 33°C.
- Dissolved oxygen (dO₂): kept above 10%.
- Duration: conducted over a period of 20 to 120 hours.

### Downstream

Upon de completion of fermentation, the oleaginous biomass is separated from the residual culture medium through a process such as centrifugation or filtration. The resulting wet biomass undergoes subsequent lysis treatments to rupture the cells and release the intracellular microbial oil. The lysis methods can be chemical, physical, enzymatic, or a combination of these.

### Chemical Lysis Method:

The biomass is chemically lysed by treating it with 1M of hydrochloric acid (HCl). The hydrolysis is conducted under the following conditions: 1.96% wt. of dry biomass/(dry biomass+HCl solution), 60°C, 2 hours and 300 rpm.

### Physical Lysis Method:

The wet biomass is subjected to high-pressure homogenizer (HPH) using the following process parameters: 1500 bars, two passes, and a wet biomass concentration of 15% wt.

### Enzymatic Lysis Method:

This method utilized commercially available enzymes, specifically those capable of breaking down polysaccharides in the cell walls. Enzymes such as mannanases (e.g., Mannaway 4.0L), β-glucanases (e.g., Ultraflo XL, Viscozyme L) and proteases (e.g., Novozym 11028) are effective in this process. These enzymes are commercially available from providers such as: Novozymes, Advanced Enzymes, ChemGen, Megazyme and many others. Optimal enzymatic lysis is achieved under the following conditions: Viscozyme L and Mannaway 4.0L enzymes, 8% by dry weight of microbial biomass, 15% enzyme-to-dry biomass ratio, pH 5.5 for Viscozyme L and pH 9 for Mannaway 4.0L, two hours of reaction (one hour per enzyme), 55°C, and 500 rpm stirring.

### Physical and Enzymatic Method:

This method combines HPH with subsequent enzymatic lysis under the following conditions:
- HPH: 1500 bars, two passes, and a wet biomass concentration of 15%.
- Enzymatic lysis: Viscozyme L, Mannaway 4.0L and Novozym 11028 enzymes, 8% by dry weight of microbial biomass, 21.8% enzyme-to-dry biomass ratio, pH 5.5 for Viscozyme L and pH 9 for Mannaway 4.0L and Novozym 11028, three hours of reaction (one hour per enzyme), 55°C, and 500 rpm stirring.

Once the cells are lysed through one or more of the aforementioned methods, the pH is readjusted to 5.5 and microbial oil is extracted through a liquid-liquid extraction process. This involves a solvent extraction where the intracellular fat is concentrated into an organic solvent phase, typically using hexane. The process consists of a 3-hour extraction with hexane, followed by an additional extraction, left to proceed 17 hours. Table 11 presents the comparative extraction yields obtained using the chemical, physical and/or enzymatic methods described above, under the same extraction conditions.

**Table 11. Solvent extraction yields achieved with different lysis methods.**

| | Extractions | |
|---|---|---|
| Lysis method | 3 hours (% wt.) | 17 hours (% wt.) |
| Chemical | 63.1 | 143.8* |
| Physical | 21.5 | 65.6 |
| Enzymatic | 19.6 | 64.5 |
| Physical and Enzymatic | 59.0 | 108.8* |

| | | |
|---|---|---|
| *>100% indicates that the solvent extracted all the fat along with additional compounds. The optimum is the closest to 100% wt. | | |

Upon completion of the extraction phase, the resulting material undergoes centrifugation at 6000 g (approximately 7360 rpm) for 10 minutes at a temperature of 20°C to separate the distinct phases:
- Organic Phase: Comprising microbial oil dissolved in hexane.
- Interphase: Containing the cellular debris in the form of a pellet.
- Aqueous Phase: Constituting the remaining liquid.

The cellular debris from the interphase is further processed to obtain yeast extract, a high-protein input for animal feed formulations, power generation applications (as it exhibits a net calorific value of 4,190 kcal/kg), and biogas production (as it yields approximately 140 L CH₄/kg).

The organic phase proceeds to the final step of the Downstream stage: solvent evaporation. In this step, the organic phase is subjected to vacuum evaporation using a rotary evaporator under the following conditions: two hours, with a cooling temperature of 5°C and bath temperature of 70°C. During this process, the hexane solvent is recovered, and the concentrated microbial oil is obtained. The recovered hexane is recirculated to the extraction step, and the microbial oil is rendered ready for commercialization.

The microbial oil obtained is considered an advanced feedstock with low carbon intensity, suitable for the production of sustainable fuels, biolubricants, and biosurfactants. This oil addresses the growing market demand for greener feedstock alternatives in both the chemical and transport sectors.

### List of Sequences

**SEQ ID NO: 1:** refers to the nucleotide sequences specific to the D1/D2 region of the 26S ribosomal DNA subunit and a fragment of the internal transcribed spacer (ITS) region, located between the 18S and 26S ribosomal DNA subunits, identified in strain L3.
**SEQ ID NO: 2:** refers to the nucleotide sequences specific to the D1/D2 region of the 26S ribosomal DNA subunit 26S and a fragment of the internal transcribed spacer (ITS) region, located between the 18S and 26S ribosomal DNA subunits, identified in strain L2.

## Claims

1. A strain of *Rhodosporidium toruloides,* deposited in the Spanish Type Culture Collection (CECT) with the accession number CECT13234, **characterized by** having the nucleotide sequence identified as SEQ ID NO: 1, or a nucleotide sequence with at least 98% identity to SEQ ID NO: 1.

2. A process for obtaining microbial oils, comprising:
a) the production of a microbial biomass with a fatty acid content equal to or greater than 40% by dry weight, by means of cultivating an oleaginous microorganism, specifically the strain CECT 13234, in a culture medium with organic waste as carbon source;
b) separation of the fatty acid content from the microbial biomass obtained in step a) to obtain microbial oils.

3. The process according to the preceding claim, wherein the organic waste is selected from the group consisting of one or more of the following: molasses, crude glycerin, lignocellulosic waste, whey, spent coffee grounds, brewery waste, pulp and paper industry effluents, food processing waste, olive mill wastewater, soybean meal, corn steep liquor, wheat straw, rice straw, corn stover, sugarcane bagasse, almond shell, olive pit, potato waste, cassava waste, fruit peels, vegetable waste, municipal waste, oat hulls and barely straw.

4. The process according to any of the preceding claims 2 or 3, wherein the carbon source is selected from the group consisting of one or more of the following: crude glycerin, and lignocellulosic sugars C5 and/or C6, preferably glucose, xylose, galactose, arabinose, and mannose.

5. The process according to the preceding claim, wherein the lignocellulosic sugars C5 and/or C6 are obtained from almond shell and/or olive pit.

6. The process according to the preceding claims 4 or 5, wherein the carbon source is crude glycerin combined with either olive pit hydrolysate or almond shell hydrolysate.

7. The process according to any of the preceding claims 4 to 6, wherein the carbon source comprises crude glycerin and olive pit hydrolysate in a glucose/xylose ratio of between 10/1 to 25/1.

8. The process according to any of the preceding claims 4 to 6, wherein the carbon source comprises crude glycerine and almond shell hydrolysate in a glucose/xylose ratio of between 10/1 to 30/1.

9. The process according to any of the preceding claims 2 to 8, wherein the carbon source comprises at least agricultural waste, and before step a), there is an upstream stage which includes the following operations: shredding, hydrothermal pretreatment, and enzymatic hydrolysis.

10. The process according to the preceding claim, further comprising a detoxification step by overliming following the enzymatic hydrolysis.

11. The process according to any of the preceding claims 2 to 10, wherein step a) is a fermentation conducted in batch mode, fed-batch mode, or a combination thereof, preferably a batch mode followed by a fed-batch mode.

12. The process according to the preceding claim, wherein the culture medium has a sugar concentration of between 30 g/L and 120 g/L upon starting the fermentation.

13. The process according to the preceding claim, wherein the culture medium comprises
NH₄NO₃ 0.1-10 g/L, CaCl₂·2H₂O 0.1-1.0 g/L, MgSO₄7·H₂O 0.1-1.0 g/L, KH₂PO₄ 0.1-1.0 g/L, Corn steep liquor (CSL) 2-10 g/L, crude glycerin and olive pit hydrolysate with a glucose/xylose ratio of 14/1 to 18/1, so the sugar concentration is between: 30-120 g/L, pH: 5.8-6.2, optionally CaCO₃ 0.1-5 g/L or
NH₄NO₃ 0.1-10 g/L, CaCl₂·2H₂O 0.1-1.0 g/L, MgSO₄7·H₂O 0.1-1.0 g/L, KH₂PO₄ 0.1-1.0 g/L, Corn steep liquor (CSL) 2-10 g/L, crude glycerin and almond shell hydrolysate with a glucose/xylose ratio of 16/1 to 23/1, so the sugar concentration is between: 30-120 g/L, pH: 5.8-6.2, optionally CaCO₃ 0.1-5 g/L.

14. The process, according to any of the preceding claims 2 to 13, wherein in step b) occurs after step a) and the microbial biomass is separated from the culture medium by centrifugation or filtration.

15. The process, according to the preceding claim, wherein the microbial biomass is lysed using chemical, and/or physical, and/or enzymatic methods, followed by a liquid-liquid extraction with an organic solvent to concentrate the microbial oil.
